# EUROPEAN PATENT APPLICATION

(11) **EP 1 170 026 A2**
(43) Date of publication of application: **09.01.2002**
(21) Application number: 01113685.0
(22) Date of filing: 20.06.2001
(51) Int. Cl.: A61M 16/06

(54) **Helmet for artificial respiration**

(30) Priority: 07.07.2000 IT MI001542
(71) Applicant: Starmed S.r.l., 41037 Mirandola (Modena) (IT)
(72) Inventor: Luppi, Libero, 41037 Mirandola (Prov. of Modena) (IT)
(74) Representative: Modiano, Guido, Dr.-Ing.

(57) **Abstract**

A helmet for artificial respiration without the aid of masks or tracheal tubes, which comprises a containment body (1), with at least one optically transparent portion (6), inside which the head of a patient can be accommodated hermetically. The containment body (1) has an air intake (10) which is connected to a ventilation apparatus and an outlet (11), the helmet also comprising a suffocation-preventing valve (20) which is suitable to connect the inside of the containment body to the outside if a pressure below a presettable value occurs inside the helmet.

## Description

The present invention relates to a helmet for artificial respiration without the aid of masks or tracheal tubes.

It is known that oxygen therapy and CPAP therapy, i.e., ventilation with continuous positive pressure, which is normally performed in intensive-case wards, pneumatology wards, infectious-disease wards, medical wards, hyperbaric chambers and so forth, currently use helmets which generally comprise a containment body provided by means of a layer of flexible plastic material which has a collar for hermetic application to the head of the patient.

The containment body is provided with an air intake, which can be connected to a ventilation machine, and with an outlet.

With these solutions it is possible to perform a noninvasive positive-pressure ventilation and respiration which is particularly useful in many fields of application.

Known types of helmets can be used at continuous positive pressure in the so-called CPAP method, and for this purpose in practice it is also possible to provide, on the air intake, a balloon made of elastically flexible material which is designed to act as a compensation element in order to maintain at all times a constant pressure level inside the helmet even during the inspiration step.

With the solutions of the prior art, therefore, it may be necessary to have a specifically provided pressure compensation element, with the obvious difficulties for connection and the presence of separate elements which can sometimes be awkward and poorly accepted by the patient.

Another drawback is that in case of malfunction of the valve assemblies or of the ventilation machine, conditions could possibly take place which are extremely dangerous for the patient, who must therefore be monitored continuously.

The aim of the invention is to eliminate the above mentioned drawbacks by providing a helmet for artificial respiration without the aid of masks or tracheal tubes which also allows to perform continuous positive pressure respiration techniques without a compensation balloon being required.

Within this aim, an object of the invention is to provide a helmet which has a very small volume, thus eliminating all operating inertias and minimizing the volume of the air during treatment.

Another object of the present invention is to provide a helmet which, thanks to its particular constructive characteristics, is capable of giving the greatest assurances of reliability and safety in use.

Another object of the present invention is to provide a helmet for artificial respiration without the aid of masks or tracheal tubes which can be easily obtained starting from commonly commercially available elements and materials and is also competitive from a merely economical point of view.

This aim and these and other objects which will become better apparent hereinafter are achieved by a helmet for artificial respiration without the aid of masks or tracheal tubes according to the invention, comprising a containment body, with at least one optically transparent portion, inside which the head of a patient can be accommodated hermetically, said containment body having an air intake which is connected to a ventilation apparatus and an outlet, characterized in that it comprises a suffocation-preventing valve which is suitable to connect the outside to the inside of said containment body if a pressure below a presettable value occurs inside said helmet.

Further characteristics and advantages of the present invention will become better apparent from the description of a preferred but not exclusive embodiment of a helmet for artificial respiration without the aid of masks or tubes, illustrated only by way of non-limitative example in the accompanying drawings, wherein:

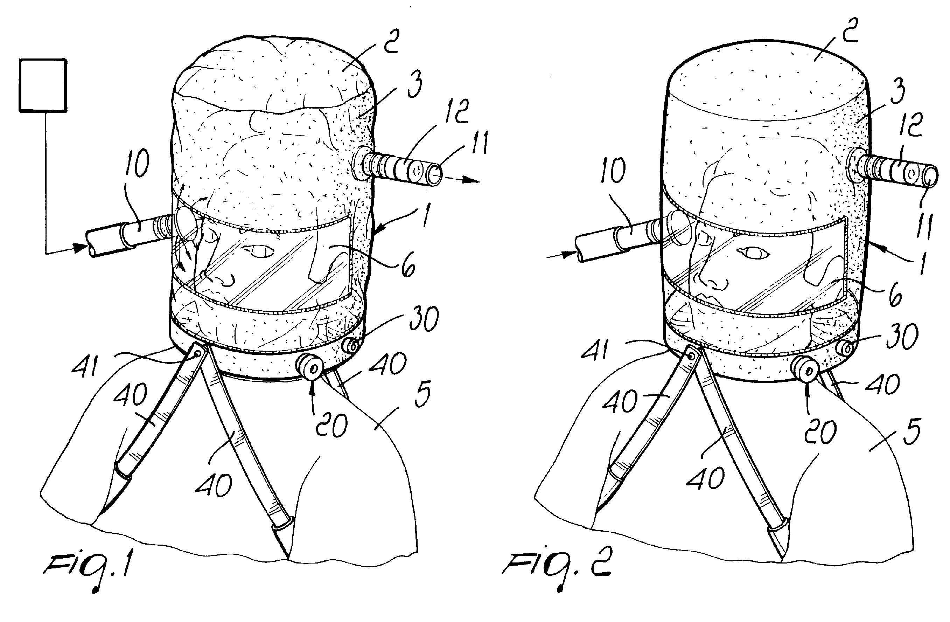

Figure 1 is a schematic perspective view of the helmet, worn by a patient;
Figure 2 is a perspective view of the helmet during the elastic expansion of the containment body;

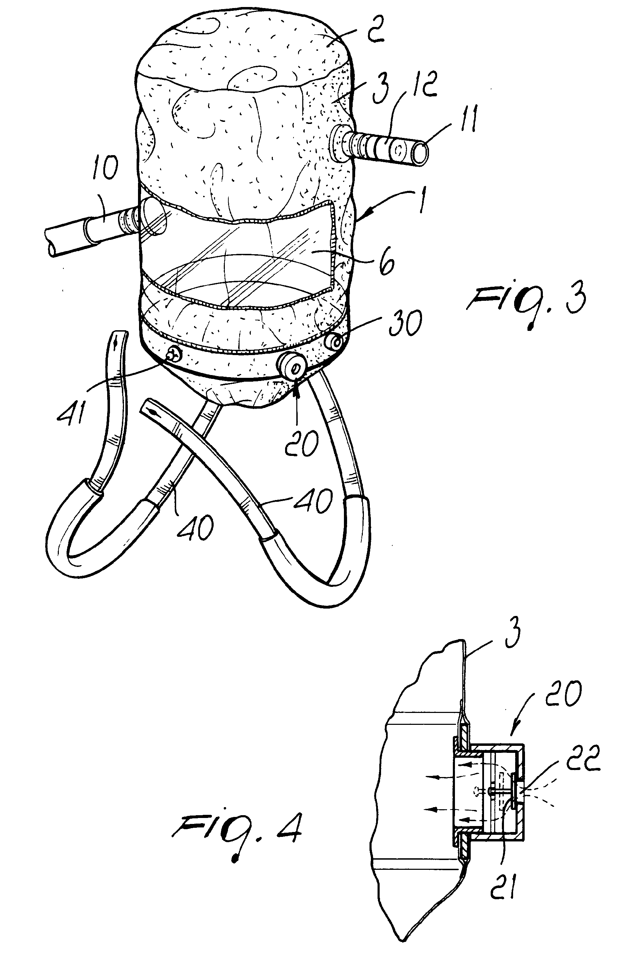

Figure 3 is a schematic perspective view of the helmet according to the invention;
Figure 4 is a view of the detail of the suffocation-preventing valve.

With reference to the figures, the helmet for artificial respiration without the aid of masks or tracheal tubes according to the invention comprises a containment body, generally designated by the reference numeral 1, which is made (this being a particular feature of the invention) of an elastically flexible material constituted by a mixture of polyurethane and PVC which forms an upper wall 2 and a side wall 3 and is connected to a lower collar 4 which is applied hermetically to the neck of the patient 5.

In order to improve comfort, on the side wall there is a mask 6 made of optically transparent material, made for example of transparent PVC, which is arranged at the front part of the face of the patient, while the remaining part is usually milky-colored and is not perfectly transparent.

An important particularity of the invention consists in that the containment body is made of elastically flexible material; accordingly, during the application of continuous positive pressure in CPAP therapy the containment body itself, by expanding elastically, is capable of acting as a compensation element without having to resort to the use of the conventional compensation balloon.

An intake 10 for introducing air and an outlet 11 for discharging the air are provided in the containment body 1.

At the intake 11, if the helmet is used with assisted pressure, it is possible to provide a pressure adjustment valve, designated by the reference numeral 12, for example of the PEEP type, which can be advantageously provided with an overpressure valve.

In constructive detail, the pressure adjustment valve is capable of adjusting the pressure for example from 0 to 20 cm of water head, while the overpressure valve that is optionally built into the control valve is set to approximately 40 cm of water head.

In order to make the helmet according to the invention particularly safe, a suffocation-preventing valve is provided, generally designated by the reference numeral 20, which is constituted in practice by a one-way valve capable of connecting the inside of the containment body to the outside when a pressure value below zero occurs inside the helmet.

In practical execution, as shown in Figure 4, it is possible to provide a membrane 21 which is normally coupled hermetically to an outlet hole 22 when there is pressure inside the helmet; as soon as the pressure is not present for any reason, the closure membrane separates from the outlet hole 22, allowing free connection to the outside.

If a maximum-pressure safety valve is not provided on the pressure control valve, then the helmet is provided with a maximum-safety pressure valve, designated by the reference numeral 30, which is advantageously set to a level of approximately 40 cm of water head.

For the sake of completeness in description, it should also be added that the helmet has two straps 40 of the underarm type which engage pins 41 formed in the containment body and are advantageously made of a soft material which prevents pressure sores and is subjected to an antibacterial treatment; said straps are designed to avoid the upward movement of the helmet due to the pressure.

From the above description it is thus evident that the invention achieves the intended aim and objects, and in particular the fact is stressed that a helmet is provided which, being made of elastically flexible material, in addition to providing an optimum seal with the neck of the user, is also capable of acting directly as a compensation balloon when the helmet is used with continuous positive pressure.

Moreover, another important aspect consists in that a suffocation-preventing valve and a maximum-pressure valve are provided which help to provide optimum helmet safety.

The invention thus conceived is susceptible of numerous modifications and variations, all of which are within the scope of the appended claims.

All the details may further be replaced with other technically equivalent elements.

In practice, the materials used, as well as the dimensions and the contingent shapes, may be any according to requirements.

The disclosures in Italian Patent Application No. MI2000A001542 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A helmet for artificial respiration without the aid of masks or tracheal tubes, comprising a containment body, with at least one optically transparent portion, inside which the head of a patient can be accommodated hermetically, said containment body having an air intake which is connected to a ventilation apparatus and an outlet, **characterized in that** it comprises a suffocation-preventing valve which is suitable to connect the inside of said containment body to the outside if a pressure below a presettable value occurs inside said helmet.

2. A helmet for artificial respiration without the aid of masks or tracheal tubes, comprising a containment body, with at least one optically transparent portion, inside which the head of a patient can be accommodated hermetically, said containment body having an air intake which is connected to a ventilation apparatus and an outlet, **characterized in that** said containment body is made of elastically flexible material in order to act as a pressure compensation element during use of the helmet with continuous positive pressure.

3. The helmet according to claim 2, **characterized in that** it comprises a lower collar which can be applied hermetically to the neck of the patient and is formed monolithically with said containment body made of elastically flexible material.

4. The helmet according to the preceding claims, **characterized in that** it comprises a maximum-pressure safety valve.

5. The helmet according to one or more of the preceding claims, **characterized in that** it comprises, on said outlet, a pressure adjustment valve with overpressure safety fixture.

6. The helmet according to one or more of the preceding claims, **characterized in that** said containment body is made of a mixture of polyurethane and polyvinyl chloride, said containment body having at least one optically transparent portion made of polyvinyl chloride.

7. The helmet according to one or more of the preceding claims, **characterized in that** it comprises means adapted to prevent the helmet from moving upwards, said means being connected to said containment body.

8. The helmet according to claim 7, **characterized in that** said means for preventing the helmet from moving upwards consists in a pair of straps which can be engaged with pins formed on said containment body and adapted to pass under the armpits of the user.
